Europäisches Patentamt

European Patent Office

Office européen des brevets

(19)

(11) Numéro de publication: **0 017 565**
**B1**

(12)

# FASCICULE DE BREVET EUROPEEN

(45) Date de publication du fascicule du brevet: **24.10.84**

(21) Numéro de dépôt: **80400409.1**

(22) Date de dépôt: **27.03.80**

(51) Int. Cl.³: **C 12 N 1/04,** C 05 F 11/08, C 12 N 11/10

(54) Procédé d'inclusion de microorganismes dans une matrice de polymère et produit ainsi obtenu.

(30) Priorité: **05.04.79 FR 7908597**

(43) Date de publication de la demande:
**15.10.80 Bulletin 80/21**

(45) Mention de la délivrance du brevet:
**24.10.84 Bulletin 84/43**

(84) Etats contractants désignés:
**FR GB IT**

(56) Documents cités:
**néant**

(73) Titulaire: **RHONE-POULENC INDUSTRIES**
**22 Avenue Montaigne**
**F-75008 Paris (FR)**

(72) Inventeur: **Jung, Gérard**
**rue des Grands Jardins**
**Leuville-sur-Orge F-91310 Montlhery (FR)**

(74) Mandataire: **Martin, Henri et al**
**RHONE-POULENC RECHERCHES Service**
**Brevets Chimie et Polymères 25, quai Paul**
**Doumer**
**F-92408 Courbevoie Cedex (FR)**

Courier Press, Leamington Spa, England.

# 0 017 565

**Description**

La présente invention a pour objet un procédé d'obtention de micro-organismes inclus dans une matrice de polymère et les produits ainsi obtenus.

Elle s'applique en particulier à l'inclusion de micro-organismes destinés à l'inoculation des légumineuses, en vue d'augmenter leur potentiel fixateur d'azote et des non-légumineuses, en vue d'améliorer leur nutrition en différents éléments.

On sait que les principales formes d'inoculation actuellement utilisées sont la troube humide ou les granulés de tourbe. De tels inoculums sont appliqués au sol ou aux graines avec divers enrobages.

On a aussi fait appel à des particules de cellulose sur lesquelles sont fixées des bactéries et un milieu de culture comme dans l'US 3 115 404, à des granulés de plâtre (FR 1 490 046) et de la lignite.

On a aussi proposé de faire appel à un gel de polymère de polyacrylamide comme dans la demande française 77 10254 du 5 Avril 1977.

Il est aussi connu d'utiliser le système enzymatique des micro-organismes inclus dans une matrice de polymères — les polymères peuvent être d'origine synthétique tels que les polyacrylamides (brevet français n° 2 320 349) ou d'origine naturelle tels que les polysaccharides (article de M. KIERSTAN paru dans Biotechnoly and Bioengineering v. 19, 1977, p. 384—397).

Dans chacune de ces publications, le micro-organisme est conservé dans un gel semi liquide n'ayant que de très faibles propriétés mécaniques et il doit être alimenté en continu pour travailler et pour survivre.

Le premier problème non résolu par l'art antérieur est celui de la survie du micro-organisme et de sa protection lors du transport et du stockage.

Un deuxième problème est l'aptitude à la libération du microorganisme inclus pour permettre son essaimage dans le milieu, et une aptitude éventuelle au greffage d'additifs. Il faut encore bien entendu assurer la pénétrabilité du milieu par les racines.

Enfin, il ne faut pas perdre de vue que le produit obtenu doit rester d'un prix suffisamment bas.

Aucune des solutions de l'art antérieur ne parvient à résoudre simultanément tous ces problèmes.

Or, la demanderesse a trouvé que l'on pouvait parvenir à un produit stable permettant la survie des micro-organismes au cours du stockage, constitué par une matrice formée par un gel de polymères à base d'au moins un polymère du groupe des polysaccharides, la matrice incluant le micro-organisme, ledit produit étant obtenu par la mise en présence des polymères en solution aqueuse et du/des micro-organisme(s) dans son milieu de culture suivie par une réticulation au moins partielle caractérisé par le fait que l'on additionne le gel obtenu d'une substance à grande capacité d'absorption d'eau et que l'on sèche de manière à obtenir une teneur en eau dans le mélange gel + absorbant supérieure à 50%.

Selon un premier mode, on forme la solution de polysaccharides à chaud avant la mise en présence des polysaccharides en solution aqueuse et du milieu de culture renfermant le micro-organisme, ou la suspension microbienne, on refroidit ensuite de manière à provoquer la réticulation. Le traitement thermique modifie la structure du polysaccharide ce qui favorise la réticulation.

Ce traitement thermique se situe préférentiellement entre 70 et 80°C. La mise en présence est alors réalisée à une température de l'ordre de 40—45°C.

Selon une autre forme de mise en oeuvre, on apporte séparément le milieu de culture ou la suspension microbienne à chaque solution de polysaccharide dans les mêmes conditions de température, on mélange et on refroidit pour former le gel.

Lorsque la solution de polysaccharides est formée à chaud on lorsque la dissolution du polysaccharide est réalisée directement dans le milieu de culture, notamment à température ambiante, la réticulation peut se faire par addition d'un sel métallique ou d'un autre polymère et de préférence d'un autre polysaccharide.

Les polysaccharides utilisés peuvent avoir des provenances diverses, soit à partir d'une fermentation microbienne, soit à partir de gommes naturelles ou biosynthétiques.

Avantageusement le polymère est à base d'un hétéro-polysaccharide à haut poids moléculaire dont l'un des polysaccharides est obtenu par fermentation d'un hydrate de carbone par un micro-organisme du genre Xanthomonas ou Arthrobacter ou des champignons appartenant au genre Sclerotium.

Les espèces représentatives de bactéries ou de champignons dont on peut se servir pour la fabrication de ces hétéropolysaccharides comprennent par exemple: Xanthomonas begoniae, le Xanthomonas campestris, le Xanthomonas carotae, le Xanthomonas hederae, le Xanthomonas incanae, le Xanthomonas malvacearum, le Xanthomonas papavericola, le Xanthomonas phaseoli, le Xanthomonas pisi, le Xanthomonas vitians, le Xanthomonas vasculorum, le Xanthomonas vesicatoria, le Xanthomonas pelgonii, l'Arthobacter stabilis, l'Arthobacter viscosus, le Sclerotium glucanicum, le Sclerotium rolfsii.

Le gel de polysaccharide peut comprendre également au moins un polysaccharide issu du groupe des gommes d'origine naturelle ou biosynthétique, de provenances diverses: algues (alginates, carraghénanes, agar), exsudats de plantes (gommes karaya, adragante, arabique), de graines (guar, caroube).

2

O 017 565

Les gommes les plus couramment utilisées sont les alginates, les kappacarraghénanes, la farine de caroube.

Si une mise en oeuvre envisagée dans le cadre de la présente invention rend nécessaire une purification du polysaccharide, on peut faire appel dans cette optique aux méthodes connues de l'art antérieur consistant par exemple à soumettre le moût fermenté, ou bien le gel aqueux reconstitué à partir de l'hétéropolysaccharide extrait du moût, à des opérations de centrifugation sur terres à diatomées, à l'action des enzymes du type protéase (cf. brevet américain n° 3 729 460).

Les micro-organismes selon l'invention sont notamment du genre Rhizobium.

Le micro-organisme est apporté de façon simple à un milieu de culture classique à base de mannitol et d'extrait de levure notamment pour le Rhizobium Japonicum.

Ce milieu de culture est ensuite mis en contact avec la solution de polysaccharides.

La concentration bactérienne dans la préparation peut être augmentée par centrifugation préalable du milieu de culture, remise en suspension du culot bactérien sous faible volume et introduction dans la solution de polysaccharide.

Ces micro-organismes peuvent être apportés seuls ou sous forme de mélanges de plusieurs genres ou espèces:

Ex: Rhizobium + levures + mycorhizes.

Pratiquement, selon un premier mode de mise en oeuvre, on forme tout d'abord une solution de polysaccharides à chaud que l'on ramène à une température de l'ordre de 40—45°C, et à laquelle on ajoute le milieu de culture renfermant le micro-organisme, ou la suspension microbienne, on refroidit ensuite de manière à former le gel.

Selon une autre forme de mise en oeuvre, on apporte séparément le milieu de culture ou la suspension microbienne à chaque solution de polysaccharide dans les mêmes conditions de température, on mélange et on refroidit pour former le gel.

On peut aussi, comme dit précédemment, faire appel à un sel métallique, tel que de fer ou d'aluminium, complexé ou non par un polyol.

De plus, on peut aussi dissoudre le polysaccharide dans le milieu de culture, notamment à température ambiante et former la réticulation in situ.

Le gel obtenu peut être conservé pendant plusieurs semaines au froid, à une température d'environ 4°C, dans une solution physiologique de NaCl.

La demanderesse a trouvé que de manière surprenante, on pouvait conserver le(s) micro-organisme(s) à la température ambiante pendant plusieurs mois en procédant à un séchage du gel.

Lors de ce séchage, il y a lieu de ne pas détruire le microorganisme, et l'on sait que celui-ci est généralement très thermosensible.

Le séchage du gel peut se faire par étalement du gel et séchage à l'air.

Un séchage tel quel est long et conduit à un film sec facilement friable et qui peut être broyé sans difficulté.

De manière préférentielle, selon l'invention, on additionne le gel d'une substance à grande capacité d'absorption d'eau poreuse, telle que silice naturelle ou synthétique, silico-aluminates, cellulose, etc. . . à un pH voisin de 7 et dans des conditions de températures suffisamment basses pour ne pas détruire le microorganisme, de l'ordre de 20 à 30°C.

La mise en forme peut être réalisée de diverses manières.

Selon un premier mode de mise en oeuvre le gel est séché, puis broyé finement, additionné d'une substance telle que la silice puis homogénéisé. La poudre ainsi obtenue peut alors être mise sous forme de pastilles.

Selon une autre forme de mise en oeuvre, on introduit le gel humide et la substance telle que la silice dans un mélangeur ou malaxeur, puis après malaxage, soit on étale et sèche directement le mélange jusqu'à ce que la perte en eau corresponde à 50% de son poids, on obtient une poudre dont la teneur en eau résiduelle est de 50 à 70 g d'eau pour 100 g de silice, soit on introduit le mélange dans une extrudeuse et on sèche les granulés obtenus à température ambiante jusqu'à également une perte en eau d'environ 50%.

De manière générale, le pourcentage d'additif peut varier de 10 à 120% selon l'additif considéré mais il est de préférence de l'ordre de 40%.

L'additif est constitué de préférence par une silice de précipitation de surface BET égale à 200 m2/g, de surface CTAB égale à 90 m2/g et de prise d'huile au dioctylphtalat égale à 340—346 cm3/100 g.

Dans tous les cas, on obtient un produit sous forme de poudre, de granulés ou de pastilles qui se prêtent à une manipulation aisée et à un bon conditionnement.

Le produit obtenu selon l'invention s'applique en particulier à l'inclusion de Rhizobium destiné à l'inoculation des légumineuses, en vue d'augmenter leur potentiel fixateur d'azote.

Le procédé selon la présente invention peut être aussi utilisé pour d'autres applications agronomiques utilisant aussi bien des bactéries que des levures ou des champignons ayant pour but l'enrichissement du sol en micro-organismes afin de favoriser:

— la détoxification des sols;

— l'oxydation ou la réduction du Fer, Soufre, Mn etc. . .

3

— la fixation d'azote atmosphérique libre ou symbiotique;
— la minéralisation de l'azote;
— l'hydrolyse de la cellulose;
— la solubilisation des phosphates;
— la dislocation des silicates etc...

La présente invention sera plus aisément comprise à l'aide des exemples suivants donnés à titre indicatif, mais nullement limitatif.

Dans une première série d'exemples on opère dans les conditions suivantes:

Le milieu de culture est constitué par le milieu YEM = Yeast Extract, Mannitol (g/l). (WACEK T. J., BRILL W. J. 1976 Crop Science $16$ 519—523).

— Mannitol 5,0—10,0; Yeast Extract Difco 0,5—1,0;
  $K_2HPO_4$ 0,5; $MgSO_4$ $7H_2O$ 0,2;
  $FeCl_3$ 0,004; NaCl 0,2;
— pH ajusté à 7,0 avec un HCl N
— $H_2O$ distillée qsp = 1000 ml
— Stérilisation: 20 min. à 120°C

*Culture liquide*
— Milieu = YEM
— Conditions: 100 ml de milieu dans un erlenmeyer de 300 ml bouché avec un tampon polyuréthane, stérilisation 20 minutes à 120°C.
— Ensemencement par une culture utilisée directement après incubation (ou éventuellement conservée à + 4°C) de Rh. japonicum souche
  $G_3$ USDA 3l1b 138 collection INRA 7, Rue Sully 21000 DIJON) sur gélose YEM ou préférentiellement par une culture inoculum liquide intermédiaire.
— Incubation 4 à 5 jours à 28°C sur table d'agitation tournant à 140 t/min., excentration du plateau 25 mm
— La population de la culture est comprise entre 1—3,0 $10^9$ germes/ml, le pH est de 7—7.2.

*Préparation du gel de polysaccharides avec Rhizobium inclus Polysaccharides utilisés.*
— Hétéropolysaccharide de type anionique, résultant de la fermentation d'hydrates de carbone par un micro-organisme de genre Xanthomonas, PM>$2.10^6$, que l'on désignera par la suite par produit A, ou simplement A.
— Farine de graine de caroube = polysaccharide constitué d'unités.

β-D-manno-pyranosyl (liaisons $1 \rightarrow 4$) une sur quatre ou cinq étant substituée en $C_6$ par un $\alpha$-D-galactopyranosyl, PM = 3,1. $10^5$, que l'on désignera par la suite par produit B ou simplement B.

*Mode opératoire*
Mode opératoire N° 1: les concentrations sont données pour la préparation de 120 g de gel:
— On mélange de poudre, 0.6 g de A + 0,6 g de B
— On amène 100 ml d'eau distillée à une T° = 70—80°C
— On verse, sous agitation, le mélange en poudre A + B
— On laisse sous agitation à une température comprise entre 70 et 80°C durant 20 à 30 minutes
— On ramène la température entre 40 et 45°C
— On ajoute 20 ml de la culture liquide
— On agite de façon à homogénéiser le mélange
— On refroidit; le gel se forme plus ou moins vite en fonction de la température de refroidissement.

Mode opératoire n° 2: Les concentrations sont données pour la préparation de 300 g de gel:
— On amène 100 ml d'eau distillée à 70—80°C, on ajoute 1,5 g de A, on laisse à cette température durant 20—30 minutes, puis on la ramène entre 40 et 45°C.
— On procéde de la même façon en remplaçant A par 1,5 g de B
— Lorsque les 2 solutions sont à 40—45°C, sous agitation, on ajoute à chacune 50 ml de la culture liquide
— On verse alors, en agitant, le mélange (culture + B) dans le mélange (culture + A)
— On refroidit

Comme précédemment on obtient un gel de consistance caoutchouteuse et le taux de survie du Rhizobium est voisin de 100%.
— Gel n° 1 6 g de gel correspondent à 1 ml de culture.
— Gel n° 2 3 g de gel correspondent à 1 ml de culture.

On observe que quel que soit le mode de préparation, le gel peut être conservé durant plusieures semaines, à 4°C dans l'eau physiologique stérile.

4

*Séchage du gel*
Mode opératoire
1) *Gel sans additif:* on écrase le gel de façon à obtenir une épaisseur maximale de 5 mm, le séchage s'effectue en 16 h—20 h, à température comprise entre 24—29°C, la perte d'eau est de 98,5% en moyenne.

2) *Gel avec additif: silice précipitée* (silice 1)
Le gel est additionné de 40% de son poids en silice précipitée de surface BET sensiblement égale à 250 m²/g prise d'huile 320 cm³/100 g. CTAB 170 m²/g, la silice est mélangée au gel jusqu'à obtention d'une poudre d'apparence légèrement humide et homogène.
Les mesures sont effectuées selon les méthodes suivantes.
Surface CTAB: surface externe par adsorption de bromure de céthyl triméthyl ammonium à pH 9 selon la méthode exposée par JAY, JANZEN et G. KRAUS dans Rubber Chemistry and Technology *44* (1971) p- 1287—1296.
La surface spécifique BET est déterminée selon la méthode de BRUNAUER — EMMETT — TELLER décrite dans The Journal of the American Chemical Society, vol. 60, p. 309, Février 1938).
Prise d'huile: au dioctylphtalate
Cette "poudre" est étalée de façon à obtenir une couche d'épaisseur comprise entre 5 et 10 mm, puis mise à sécher entre 24—29°C jusqu'à ce que la perte en eau corresponde à 50—55% du poids du mélange; on obtient alors une poudre facilement manipulable apparemment sèche dont la teneur en eau résiduelle est de 50 à 70 g par 100 g de silice additionnés. Dans ces conditions, le séchage dure de 7 à 8 h.

Résultats
*Viabilité de Rhizobium japonicum après séchage*
Afin de mettre en évidence l'effet protecteur du gel sur la survie du micro-organisme, on ajoute la silice soit à la culture seule, soit à la culture additionnée de 10 g/l d'hétéropolysaccharide soit à des gels préparés selon l'invention, à raison de 40 g par 100 g de la culture ou du mélange considéré.
Ces préparations qui ont l'aspect d'une "poudre humide" sont séchées durant des intervalles de temps variables de façon à ce que la teneur en eau résiduelle des poudres s'échelonne entre 150 et 0% (= dessiccation). Les résultats obtenus sont donnés dans le tableau 1.
Parallèlement on indique les resultats obtenus avec le gel séché sans additif ou en présence d'un additif inerte (sable ou poudre de verre).
La viabilité du micro-organisme est déterminée par la technique classique des suspensions dilutions étalées sur milieu gélosé YEM.
Il est également possible d'obtenir une poudre prête à l'emploi en mélangeant le gel avec 65—100% de son poids en silice.

TABLEAU I

Viabilité de Rh. japonicum au cours du séchage
(log nb. germes rapporté à 1 ml de culture)

| H$_2$O residuelle (% additif*) / Traitement | 140 | 100 | 50 | 10 | Dessiccation | |
|---|---|---|---|---|---|---|
| | | | | | 10—20 h | 25—30 h |
| Culture — silice 1 | 8,0 | 7,9 | 7,6 | 6,4 | 5,0 | 3,6 |
| Culture + Produit selon l'invention et silice | 8,7 | 8,6 | 7,9 | 6,7 | 5,6 | 4,7 |
| Gel (No 2) — silice 1 | 9,0 | 8,8 | 8,7 | 7,9 | 7,4 | 6,9 |
| Gel + Cellulose | | | | 8,3 | 7,3 | 5,3 |
| Gel + poudre de verre | | | | | 7,8 | 7,6 |
| Gel + sable | | | | | 8,3 | |
| Gel (no 2) | | | | | 8,3 | |
| Gel (no 1) | | | | | 7,5 | |

*Additif = 40% du poids de la culture ou du gel (sauf pour le sable = 60%).

On observe que le procédé selon l'invention met en évidence après séchage:
— un effet protecteur du gel,
— une bonne survie du micro-organisme dans le gel additionné ou non d'une substance inerte.
Mais l'addition de silice dans ce cas n'est compatible avec une bonne survie du micro-organisme que si la teneur en H$_2$O résiduelle est supérieure à un seuil de l'ordre de 50% du poids d'additif, voir tableau II.
De manière générale, le pourcentage d'additif peut varier de 10 à 120% selon l'additif considéré mais est de préférence de l'ordre de 40%.

TABLEAU II

VIABILITE DE Rh. JAPONICUM AU COURS DU STOCKAGE A 28°C
EN FONCTION DE LA TENEUR INITIALE EN EAU RESIDUELLE
(MELANGE GEL + SILICE RESULTATS EXPRIMES EN log nb germes)

| H$_2$O résiduelle initiale (% silice) | log. nb. germes (rapportés à 1 ml de culture) | |
|---|---|---|
| | temps 0 | après 13 j à 28°C |
| 155 | 8,5 | 8,4 |
| 78 | 8,5 | 7,4 |
| Dessiccation | 6,8 | 3,0 |

*Viabilité du Rhizobium japonicum au cours du stockage à 28°C*

Les échantillons déshydratés (gels tels que ou avec additifs inertes) ou renfermant une certaine teneur en eau (gel + silice) sont conservés à 28°C en flacon bouché durant des intervalles de temps variables.

Les résultats obtenus:

— sur le gel déshydraté

— sur le gel + silice renfermant 56 à 81% d'eau résiduelle, sont représentés dans le tableau III.

On note une survie du micro-organisme satisfaisante pendant au moins 60—70 jours.

TABLEAU III

Viabilité de Rh. japonicum au cours du stockage à 28°C (log nb. germes)

| Echantillon | H₂O résiduelle | nb jours stockage 28°C | nb essais | log nb germes (rapportés à 1 ml de culture) | |
|---|---|---|---|---|---|
| | | | | moyenne | Valeurs extrêmes |
| Gel (n° 1 ou 2) | < 1 | 0 | 9 | 8,0 | 7,2 — 8,6 |
| | | 7 — 14 | 6 | 7,3 | 6,4 — 8,2 |
| | | 17 — 25 | 3 | 7,5 | 6,9 — 7,9 |
| | | 34 — 35 | 3 | 6,6 | 5,6 — 7,7 |
| | | 68 | 2 | 6,9 | 6,7 — 7,1 |
| Gel (n° 1 ou 2) silice 1 | 56 — 81 | 0 | 12 | 8,6 | 8,5 — 8,7 |
| | | 4 — 7 | 5 | 8,1 | 7,2 — 8,6 |
| | | 9 — 15 | 8 | 7,4 | 6,4 — 8,5 |
| | | 21 — 25 | 5 | 7,6 | 6,9 — 8,1 |
| | | 45 | 1 | 5,9 | — |
| | | 60 | 1 | 8,3 | — |

L'infectivité de Rh. japonicum a été contrôlé sur Clycine max. variété AMSOY 71 en milieu gélosé synthétique sans azote selon un protocole dérivé de celui décrit par VINCENT J. M. 1970.

Le nombre de nodules formés à partir d'un inoculum constitué par la culture ou le mélange (gel + silice) séché et stocké à 28°C (durant 4—27 ou 60 jours) est respectivement de 56 et 55, donc il y a conservation de la viabilité et de l'infectivité du micro-organisme dans les inoculums ainsi préparés.

De plus la présente invention permet des mises en forme en pastilles ou granulés.

1) *Gel sans additif* après séchage, le gel est broyé finement, additionné de silice puis homogénéisé. La poudre ainsi obtenue est pastillée sans difficulté dans une pastilleuse de type ARCA; on obtient alors des pastilles sèches de forme cylindrique ($\emptyset$ = 3 mm h = 3 mm).

2) *Gel avec additif:* le gel humide puis la silice sont introduits dans un mélangeur de type KUSTNER, et malaxés durant 5 à 10 mn; le mélange ainsi obtenu est traité de 2 façons différentes:

— soit étalement et séchage direct du mélange entre 24—29°C jusqu'à ce que la perte en eau corresponde à 50% de son pids, puis mise en forme dans une pastilleuse,

— soit préférentiellement introduction du mélange dans une "extrudeuse", de type ALEXANDERWERK, d'où obtention de granulés humides ($\emptyset$ = 3 mm, h = 3 mm); ces granulés sont séchés très rapidement à température ambiante jusqu'à perte de 50% de leur poids; le séchage est accéléré par passage d'un flux d'air sur ces préparations.

Dans tous les cas, on obtient des granulés résistants qui se prêtent facilement à la manipulation ou au conditionnement.

Par ailleurs, l'on peut réaliser une réticulation par un sel métallique de fer ou d'aluminium complexé ou non par un polyol (sorbitol, glycérol). Cette solution de sel métallique est additionnée dans un milieu de culture contenant 5 à 15 g/l du même hétéropolysaccharide que précédemment.

Dans le cas de l'aluminium, on a mis en oeuvre du sulfate et du nitrate et préférentiellement du chlorure. On a ainsi pu obtenir des fibres avec perte en eau par synerese comprise entre 80 et 90% du poids total initialement mis en oeuvre.

7

**0 017 565**

Dans une seconde série d'exemples on réalise des préparations à base de carraghénane (kappa) et d'alginate.

Préparation des gels avec Rhizobium inclus

*Polysaccharides utilisés*

*Kappa-carraghénane:* unités galactose plus ou moins sulfatés alternativement liées en 1—3 ou 1—4 (unité D-galactopyranose 4 sulfate liaison $1 \to 3 + 3$—6 anhydro D-galactopyranose liaison $1 \to 4$).

*Alginate de sodium:* copolymère d'unités d'acides $\beta$-D-($1 \to 4$) mannopyranosyluronique et d'acides $\alpha$-L-($1 \to 4$) gulopyranosyluronique, dont le rapport varie en fonction de l'espèce algale utilisée; PM = 32 à $200.10^3$ selon le degré de polymérisation.

Mode opératoire

*Préparation à base de Kappa-carraghénane* (Kc)

Les concentrations sont données pour la préparation de 160 g de gel.

Mode opératoire n° 1 (gel Kc)

On amène 110 ml d'eau distillée à T° = 70°—80°C,

On verse sous agitation 1,6 g de poudre Kc,

On laisse sous agitation à une température voisine de 70°C jusqu'à dissolution complète (20 minutes environ),

On ramène la température à $\sim 45°C$,

On ajoute 50 ml de la culture liquide, sous agitation, jusqu'à obtention d'un mélange homogène.

Le pH du mélange obtenu est alors compris entre 7 et 7,5; le gel se forme plus ou moins vite selon la température de refroidissement: 60 à 90 minutes à température ambiante.

Mode opératoire n° 2 (gel Kc—Ca)

On opère comme précédemment mais au moment d'additionner la culture liquide on rajoute à cette dernière 0,8 g de $CaCl_2$, $2H_2O$.

Le pH est alors compris entre 6,5 et 7,0 et le gel se forme en 5 à 15 minutes à température ambiante.

Mode opératoire n° 3 (gel Kc—Gc)

On mélange en poudre 1,2 g de poudre Kc et 0,4 g de farine de graine de caroube,

On amène 110 ml d'eau distillée à 70—80°C,

On verse, sous agitation, le mélange des poudres,

On laisse sous agitation entre 70—80°C durant 20 minutes environ,

On ramène la température à $\sim 45°C$.

On ajoute 50 ml de la culture liquide, sous agitation, de façon à obtenir un mélange homogène.

Le pH du mélange est compris entre 7,2 et 7,5 et le gel se forme en 30—40 minutes à température ambiante.

*Preparations à base d'alginate de sodium*

Les concentrations sont données pour la préparation de 100 g de gel; toutes les opérations s'effectuent à température ambiante et sous agitation.

Mode opératoire n° 4 (gel ALG—Ca)

On ajoute 1 g d'alginate de sodium (alginate haute viscosité S 800 établissements François), à 80 ml de culture liquide,

Après dissolution et homogénéisation on additionne 20 ml de solution de $CaSO_4$, $2H_2O$ à 6 g/l.

Le pH du mélange est compris entre 7,0 et 7,5, la gélification est presque instantanée.

Mode opératoire n° 5 (gel ALG—Ca—$PO_4$)

On opère comme précédemment mais les 20 ml de solution de $CaSO_4$, $2H_2O$ contiennent en outre $Na_2HPO_4$ à la concentration de 1 g/l.

Le pH est voisin de 7,5; la gélification est retardée par l'addition de $Na_2HPO_4$, 5 à 10 minutes.

Ces modes opératoires ne sont pas limitatifs. Des gels avec prises en masse plus ou moins rapides peuvent être obtenus en modifiant la concentration des polysaccharides et des sels, la température de préparation, la nature et la valence des cations utilisés.

La viabilité du micro-organisme dans toutes ces préparations non déshydratées est de 100%.

*Séchage du gel*

Le protocole de séchage est identique à celui des exemples précédents. Divers additifs de séchage sont utilisés:

8

— silice synthétique: (2) CTAB 90 m²/g, BET 200 m²/g — prise d'huile 346 cm³/100 g
— silice synthétique: (3) CTAB 90 m²/g, BET 200 m²g — prise d'huile 340 cm³/100 g
— silice naturelle CLARCEL FLO silice (4) à base de Perlite (Ets CECA) — prise d'huile 350 cm³/100 g.

Résultats

*Viabilité de Rhizobium japonicum inclus après séchage et stockage à 28°C*
*Gel sans additif*

On a comparé l'influence de divers polysaccharides, seuls, en mélange ou avec addition de sels sur la survie de 2 souches de *Rhizobium japonicum* (souches $G_3$ (USDA 3I1 b 138) et $G_2SP$ (UDSA 3I1 b 125) collection INRA DIJON) après inclusion, déssiccation et stockage à 28°C.

| Gel déshydraté | Poids gel sec correspondant à 1 ml culture (mg) | VIABILITE (log nb germes rapporté à 1 ml de culture) | | | |
|---|---|---|---|---|---|
| | | souche G3 | | souche G2SP | |
| | | t = 0 | t = 60j | t = 0 | t = 50j |
| gel 2 — [1] | 40 | 8,9 | 8,7 | 7,7 | 0 |
| Kc | 38 | 8,6 | 7,6 | 6,7 | 0 |
| Kc–Ca | 66 | 8,4 | 6,5 | – | 0 |
| Kc–Gc | 36 | 9,2 | 8,7 | 6,7 | 6,7 |
| ALG–Ca | 27 | 9,1 | 8,5 | 8,1 | 8,1 |
| ALG–Ca–PO₄ | 22 | 8,8 | 8,1 | 7,7 | 7,2 |
| PPA[2] | 80 | 7,8 | 5,5 | 6,1 | 0 |

[1] gel gomme Xanthane + farine graine caroube
[2] gel polyacrylamide (décrit dans le brevet 77 10254 du 5/4/77).

Il apparait que pour une espèce de micro-organisme donnée la résistance à la dessiccation peut être différente selon la souche considérée; le type de polymère servant à l'inclusion joue un rôle important.

*Gel avec additif*

Le gel est additionné de 40% de son poids, soit de silice précipitée, soit de silice naturelle (CLARCEL FLO à base de Perlite); après mélange on évapore jusqu'à obtention d'une poudre dont la teneur en eau résiduelle est comprise entre 0 et 75 g par 100 g d'additif; les échantillons sont conservés de préférence en sacs ou flacons hermétiquement clos à une température de 28°C.

*Influence de l'additif sur la survie du Rhizobium*

Un gel à base de gomme xanthane + farine de graine de caroube est additionné de silice synthétique ou naturelle, séché jusqu'à ce que la teneur en eau résiduelle soit comprise entre 50 et 70% la viabilité de *Rh. japonicum* (souche $G_3$) est voisine de 100% après 8 mois de conservation à 28°C pour gel + additif alors qu'elle est pratiquement nulle pour le gel déshydraté sans additif.

9

| Nb. jours stockage à 28°C | poudre sèche gel 2 (1) | VIABILITE (log nb germes rapporté à 1 ml) de culture) | | |
|---|---|---|---|---|
| | | poudre à 50–70% eau résiduelle | | |
| | | gel 2 + silice 2 | gel 2 + silice 3 | gel 2 + silice naturelle |
| 0 | 8,7 | 8,7 | 9,2 | 9,2 |
| 30–40 | 7,9 | 8,8 | 9,0 | 9,4 |
| 80 | 7,0 | | 8,8 | |
| 120 | 6,5 | | 9,0 | |
| 160–175 | 5,5 | 8,3 | 8,9 | |
| 210–220 | 4,8 | 8,2 | 9,1 | |
| 250 | 4,0 | 7,4 | | |
| 300 | ⩽3 | | | 9,2 |

(1) valeurs moyennes de 10 essais.

*Influence de la nature de l'additif et de la teneur en eau résiduelle sur la survie de Rh. japonicum (souche $G_3$)*

Le gel est additionné de silice 1, 2, 3 ou CLARCEL FLO et les mélanges obtenus sont séchés selon le cas jusqu'à une teneur en eau résiduelle comprise entre 0 et 75%.

| Nb. jours stockage à 28°C | gel (2) + additif | $H_2O$ résiduelle (% additif) | | | | | |
|---|---|---|---|---|---|---|---|
| | | 75 | 50 | 36 | 25 | 10 | 0 |
| 0 | silice 1 | 8,7 | 8,7 | | | 7,9 | 6,9 |
| | ,, 2 | 8,8 | | 9,1 | | | 8,3 |
| | ,, 3 | 9,1 | | 9,1 | | | 8,3 |
| | ,, 4 | 9,4 | 9,2 | 9,0 | 9,4 | 8,8 | 8,5 |
| 12–28 | silice 1 | 7,8 | 7,4 | | | ⩽3 | <3 |
| | ,, 2 | 8,7 | | 8,5 | | | 7,4 |
| | ,, 3 | 8,8 | | 8,6 | | | 7,4 |
| | ,, 4 | 9,4 | 9,4 | 9,6 | 9,2 | 8,4 | 4,6 |
| 40–62 | silice 1 | 6,6 | | | | | |
| | ,, 2 | 8,5 | | 8,5 | | | 6,7 |
| | ,, 3 | 8,6 | | 8,4 | | | 6,3 |
| | ,, 4 | 9,3 | 9,6 | 9,6 | 9,4 | 8,8 | 3,8 |
| 220–300 | silice 1 | 3,0 | | | | | |
| | ,, 2 | 7,4 | | | | | |
| | ,, 3 | 9,1 | | | | | |
| | ,, 4 | | 9,2 | 9,4 | 8,8 | 8,6 | < 3 |

Alors que l'additif constitué par de la silice 1 ne permet qu'une survie limitée de *Rhizobium* dans le temps même lorsque la teneur en eau résiduelle est élevée (~75%), en revanche avec les additifs 2 et 3 la survie du micro-organisme est de 100% même lorsque la teneur en eau résiduelle est comprise entre 10 et 30%.

*Effet protecteur de l'additif au cours du stockage à 28°C*

4 souches de *Rh. leguminosarum* (souches PD12, 1007, FH13, FH20S1 collection INRA. 7 rue Sully 21000 DIJON) sensibles à la dessiccation ont été incluses dans du gel de gomme xanthane + farine de graine de caroube (2) et l'inoculum ainsi préparé a été additionné de silice à forte rétention d'eau (1 ou 2), après séchage (jusqu'à 75% d'eau résiduelle) les échantillons ont été conservés en flacon bouché à 28°C.

| Souche Rh. leguminosarum (1) | Viabilité (log. nbg. rapporté à 1 ml culture) | | | | | |
|---|---|---|---|---|---|---|
| | Gel 2 déshydrate | | Gel 2 + silice 1 | | Gel 2 + silice 2 | |
| | 0 | 30 j. | 0 | 30 j. | 0 | 30 j. |
| PD 12 | 8,2 | <5 | 8,6 | 7,3 | 8,6 | 8,3 |
| 1007 | 8,5 | 6,6 | 8,6 | 5,8 | 9,0 | 9,1 |
| FH 13 | 8,7 | <5 | 8,9 | 7,5 | 8,4 | 8,8 |
| FH 20S1 | 8,9 | 6,3 | 8,4 | 7,0 | 8,7 | 8,6 |

(1) milieu de culture (g/l): saccharose 10 — $K_2HPO_4$ 0,5 — $MgSO_4$ 7 $H_2O$ 0,2 — NaCl 0,2 — $FeCl_3$ 0,004 Yeast Extract Difco 1 — $CaCO_3$ 2 à 5.

Il n'y a que dans les préparations additionnées de silice 2 que la viabilité est de 100% après 30 jours de conversion.

Les exemples précédents montrent tout l'intérêt de la présente invention. Bien évidemment l'inoculation des graines ou du sol peut se faire de toute manière connue, telle, par exemple que décrit dans l'exposé de l'art antérieur.

**Revendications**

1. Procédé d'obtention de micro-organismes inclus dans une matrice constituée par un gel de polymères à base d'au moins un polymère du groupe des polysaccharides par mise en présence des polymères en solution aqueuse et du (des) micro-organisme(s) dans son milieu de culture suivi par une réticulation au moins partielle, caractérisé en ce qu'on additionne le gel obtenu d'une substance à grande capacité d'absorption d'eau et que l'on sèche, de manière à obtenir une teneur en eau dans le mélange gel + absorbant supérieure à 50%.

2. Procédé selon la revendication 1, caractérisé en ce que l'on forme une solution de polysaccharides à chaud avant la mise en présence du polysaccharide en solution aqueuse et des micro-organismes dans leur milieu de culture.

3. Procédé selon la revendication 2, caractérisé en ce que le chauffage est réalisé entre 70 et 80°C.

4. Procédé selon les revendications 2 ou 3, caractérisé en ce que la mise en présence des polymères et du (des) micro-organisme(s) est réalisée à une température de l'ordre de 40 à 45°C.

5. Procédé selon les revendications 2 et 3, caractérisé en ce que l'étape de réticulation est réalisée par refroidissement.

6. Procédé selon la revendication 1, caractérisé en ce que l'étape de réticulation est réalisée par addition de sel métallique tel que de fer ou d'aluminium, complexé ou non par un polyol.

7. Procédé selon les revendications 1 ou 6, caractérisé en ce que la mise en présence se fait à température ambiante.

8. Procédé selon l'une quelconque des revendications précédentes, caractérisé en ce que le polymère est à base d'un hétéropolysaccharide à haut poids moléculaire dont l'un des polysaccharides est obtenu par fermentation d'un hydrate de carbone par une bactérie du genre Xanthomonas ou Arthrobacter ou par un champignon appartenant au genre Sclérotium.

9. Procédé selon la revendication 7, caractérisé en ce que le gel de polysaccharide comprend également au moins un autre polysaccharide du groupe des gommes d'origine naturelle.

10. Procédé selon la revendication 9, caractérisé en ce que les polysaccharides sont choisis parmi: les alginates, les kappa carraghénanes, la farine de caroube.

11. Procédé selon les revendications 1 ou 4, caractérisé en ce que l'on apporte séparément le milieu de culture ou la suspension microbienne à chaque solution de polysaccharide.

11

12. Procédé selon l'une quelconque des revendications précédentes, caractérisé en ce que le micro-organisme est du genre Rhizobium.

13. Procédé selon la revendication 1, caractérisé en ce que le séchage se fait à l'air.

14. Procédé selon la revendication 1, caractérisé par le fait que le produit additionné est constitué par de la silice de manière à ce que la teneur en eau résiduelle du mélange gel + silice soit de 50 à 70 g par 100 g de silice.

15. Procédé selon l'une des revendications 1 et 14, caractérisé par le fait que le pourcentage d'additif est de 10 à 120% en poids par rapport au gel.

16. Procédé selon l'une des revendications 1 et 14, caractérisé par le fait que l'additif est constitué par une silice de précipitation de surface BET égale à 200 m2/g, de surface CTAB égale à 90 m2/g et de prise d'huile au dioctylphtalate égale à 340—346 cm3/100 g.

17. Micro-organisme inclus dans une matrice de polymère, caractérisé par le fait qu'il est obtenu en mettant en oeuvre le procédé selon l'une des revendications 1 à 16.

## Claims

1. A process for producing micro-organisms which are included in a matrix formed by a gel of polymers based on at least one polymer of the polysaccharides group by bringing together the polymers in aqueous solution and the micro-organism(s) in the culture medium thereof; followed by at least partial cross-linking, characterised in that added to the gel produced is a substance having a high water absorption capacity, and that drying is effected so as to achieve a water content in the gel + absorbing agent mixture that is higher than 50%.

2. A process according to claim 1 characterised by forming a solution of polysaccharides in the hot condition before bringing together the polysaccharide in aqueous solution and the micro-organisms in their culture medium.

3. A process according to claim 2 characterised in that the heating operation is carried out at between 70 and 80°C.

4. A process according to claim 2 or claim 3 characterised in that the polymers and the micro-organism(s) are brought together at a temperature of the order of from 40 to 45°C.

5. A process according to claim 2 and claim 3 characterised in that the cross-linking step is effected by cooling.

6. A process according to claim 1 characterised in that the cross-linking step is effected by adding a metal salt such as of iron or aluminium, which may or may not be chelated by a polyhydric alcohol.

7. A process according to claim 1 or claim 6 characterised in that the operation of bringing the substances together is effected at ambient temperature.

8. A process according to any one of the preceding claims characterised in that the polymer is based on a heteropolysaccharide having a high molecular weight, wherein one of the polysaccharides is produced by fermentation of a carbohydrate by a bacterium of the genus Xanthomonas or Arthrobacter or by a fungus belonging to the genus Sclerotium.

9. A process according to claim 7 characterised in that the polysaccharide gel also comprises at least one other polysaccharide from the group of gums of natural origin.

10. A process according to claim 9 characterised in that the polysaccharides are selected from: alginates, kappa carragheenanes and carob flour.

11. A process according to claim 1 or claim 4 characterised in that the culture medium or the microbial suspension is added separately to each polysaccharide solution.

12. A process according to any one of the preceding claims characterised in that the micro-organism is of the genus Rhizobium.

13. A process according to claim 1 characterised in that the drying operation is carried out in air.

14. A process according to claim 1 characterised in that the added substance comprises silica so that the proportion of residual water in the gel + silica mixture is from 50 to 70 g per 100 g of silica.

15. A process according to one of claims 1 and 14 characterised in that the percentage of additive is from 10 to 120% by weight with respect to the gel.

16. A process according to one of claims 1 and 14 characterised in that the additive comprises a precipitation silica with a BET surface area of 200 m$^2$/g, a CTAB surface area of 90 m$^2$/g, and dioctylphthalate oil absorption of 340—346 cm$^3$/100 g.

17. A micro-organism included in a polymer matrix characterised in that it is produced by carrying out the process according to one of claims 1 to 16.

## Patentansprüche

1. Verfahren zum Erhalten von Mikroorganismen, die in eine Matrix, bestehend aus einem Gel von Polymeren auf der Basis mindestens eines Polymeren aus der Gruppe der Polysaccharide eingeschlossen sind, durch Zusammenbringen von Polymeren in wäßriger Lösung und des (der) Mikroorganismus(men) in seinem (ihrem) Kulturmedium und anschließende, zumindest partielle Vernetzung, dadurch gekennzeichnet, daß man zu dem erhaltenen Gel eine Substanz mit großer Wasserabsorptionskapazität gibt und so trocknet, daß man einen Wassergehalt in dem Gemisch aus Gel und Absorptionsmittel von mehr als 50% erhält.

2. Verfahren nach Anspruch 1, dadurch gekennzeichnet, daß man eine heiße Polysaccharidlösung herstellt, bevor man die wäßrige Polysaccharidlösung und die Mikroorganismen in ihrem Kulturmedium zusammenbringt.

3. Verfahren nach Anspruch 2, dadurch gekennzeichnet, daß man auf 70 bis 80°C erhitzt.

4. Verfahren nach Anspruch 2 oder 3, dadurch gekennzeichnet, daß man die Polymeren und den (die) Mikroorganismus(men) bei einer Temperatur im Bereich von 40 bis 45°C zusammenbringt.

5. Verfahren nach den Ansprüchen 2 und 3, dadurch gekennzeichnet, daß man die Stufe der Vernetzung durch Abkühlen ausführt.

6. Verfahren nach Anspruch 1, dadurch gekennzeichnet, daß man die Stufe der Vernetzung durch Zugabe eines Metallsalzes, beispielsweise von Eisen oder Aluminium, mit einem Polyol in einen Komplex überführt oder nicht, ausführt.

7. Verfahren nach den Ansprüchen 1 oder 6, dadurch gekennzeichnet, daß das Zusammenbringen bei Raumtemperatur erfolgt.

8. Verfahren nach einem der vorangehenden Ansprüche, dadurch gekennzeichnet, daß das Polymer (ein Polymer) auf der Basis eines hochmolekularen Heteropolysaccharids ist, von dessen Polysacchariden eines durch Fermentation eines Kohlenhydrats mit einem Bakterium der Gattung Xanthomonas oder Arthrobacter oder durch einen Pilz der Gattung Sclerotium erhalten worden ist.

9. Verfahren nach Anspruch 7, dadurch gekennzeichnet, daß das Polysaccharidgel auch mindestens ein weiteres Polysaccharid aus der Gruppe der Gummen natürlicher Herkunft umfaßt.

10. Verfahren nach Anspruch 9, dadurch gekennzeichnet, daß die Polysaccharide ausgewählt werden unter: den Alginaten, kappa-Carraghenanen und Karubamehl.

11. Verfahren nach den Ansprüchen 1 oder 4, dadurch gekennzeichnet, daß man das Kulturmedium oder die Mikrobensuspension getrennt zu jeder Polysaccharidlösung zugibt.

12. Verfahren nach einem der vorangehenden Ansprüche, dadurch gekennzeichnet, daß der Mikroorganismus zur Gattung Rhizobium gehört.

13. Verfahren nach Anspruch 1, dadurch gekennzeichnet, daß das Trocknen an der Luft erfolgt.

14. Verfahren nach Anspruch 1, dadurch gekennzeichnet, daß das zugesetzte Produkt aus Kieselsäure besteht derart, daß der Restwassergehalt des Gemisches aus Gel und Kieselsäure 50 bis 70 g je 100 g Kieselsäure ausmacht.

15. Verfahren nach einem der Ansprüche 1 und 14, dadurch gekennzeichnet, daß der prozentuale Anteil des Zusatzes 10 bis 120 Gew.-%, bezogen auf das Gel, ausmacht.

16. Verfahren nach einem der Ansprüche 1 und 14, dadurch gekennzeichnet, daß der Zusatz aus einer Fällungskieselsäure mit einer BET-Oberfläche von 200 $m^2/g$, einer CTAB-Oberfläche von 90 $m^2/g$ und einer Ölaufnahme für Dioctylphthalat von 340 bis 346 $cm^3/100$ g besteht.

17. Mikroorganismus, eingeschlossen in eine Polymermatrix, dadurch gekennzeichnet, daß er durch Ausführung des Verfahrens nach einem der Ansprüche 1 bis 16 erhalten worden ist.